# EUROPÄISCHE PATENTSCHRIFT

(11) **EP 1 566 170 B1**
(45) Veröffentlichungstag und Bekanntmachung des Hinweises auf die Patenterteilung: **05.09.2007**
(21) Anmeldenummer: 05100322.6
(22) Anmeldetag: 20.01.2005
(51) Int. Cl.: A61K 8/63, A61K 8/97, A61Q 19/00, A61Q 19/08

(54) **Hautpflegeprodukt enthaltend Ursolsäure und Ginkgo-Extrakt**
Product for skin care containing ursolic acid and gingko extract
Produit pour le soin de la peau contenant acide ursolique et un extrait de gingko

(30) Priorität: 20.02.2004 DE 102004009155
(43) Veröffentlichungstag der Anmeldung: 24.08.2005
(73) Patentinhaber: Beiersdorf AG, 20253 Hamburg (DE)
(72) Erfinder: Kröpke, Rainer, 22869, Schenefeld (DE); Filbry, Alexander, 22459 Hamburg (DE); Heinecke, Silke, 21031 Hamburg (DE); Lindemann, Wiebke, 20257, Hamburg (DE)

(56) Entgegenhaltungen:
- WO-A-02/15869
- DE-C1- 4 429 928
- FR-A- 2 699 818
- US-A1- 2002 028 257
- US-A1- 2002 192 245
- US-B1- 6 207 711

## Beschreibung

Die vorliegende Erfindung betrifft ein Hautpflegeprodukt enthaltend Salze der Ursolsäure und Ginkgo-Extrakt.

Die Haut ist unser größtes Organ und gleichzeitig Spiegelbild unserer Seele. Kein anderes Organ ist äußeren Einflüssen so stark ausgesetzt. Hitze, Kälte oder Sonneneinstrahlung, falsche oder übertriebene Hautreinigung - das alles belastet die Haut.

Jeden Tag verdunstet dabei über die Hautoberfläche Wasser. Bei Hauttrockenheit ist das Gleichgewicht zwischen Nachschub und Verdunstung gestört, so dass der Feuchtigkeitsverlust überwiegt - die Haut trocknet aus. Innere und äußere Faktoren beeinflussen den Grad der Austrocknung zusätzlich, wie hormonelle Einflüsse, die biologische Hautalterung, Krankheiten sowie Ernährung, Licht, Umwelt und Klima.

Kosmetische Hautpflege dient in erster Linie dazu, die natürliche Funktion der Haut als Barriere gegen Umwelteinflüsse (z. B. Schmutz, Chemikalien, Mikroorganismen) und gegen den Verlust von körpereigenen Stoffen (neben Wasser auch natürliche Fette, Elektrolyte etc.) zu stärken oder wieder herzustellen.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen, Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z. B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z. B. Rissigkeit).

Es sind eine Vielzahl von Produkten und Wirkstoffen zur Pflege der Haut bekannt. Retinol, Vitamin A, wirkt beispielsweise gegen übermäßige Verhornung und Pigmentierung der Haut. Ein Mangel an Vitamin A äußert sich deshalb in trockener, rauher, verhomter Haut und einer Atrophie der Schweißdrüsen. Der Zusatz von Vitamin A zu Kosmetika ist insbesondere bei der Behandlung alternder Haut, bei der eine stärkere Verhornung vorliegt, indiziert. Vitamin A ist somit auch ein kosmetischer Wirkstoff zur prophylaktischen Aknebehandlung. Vitamin A ist allerdings auch empfindliche gegen Luft, Wärme und vor allem Lichteinflüssen.

Liponsäure, Coenzym Q10 sind weitere bekannte Wirkstoffe, die nachgewiesenermaßen eine pflegende und wiederaufbauende Wirkung insbesondere alternder Haut zeigen. Diese Wirkstoffe werden auch als Antioxidantien d.h. als Schutzsubstanzen gegen den Verderb der sie enthaltenden Zubereitungen verwendet. Dennoch ist bekannt, dass auch in der menschlichen und tierischen Haut unerwünschte Oxidationsprozesse auftreten können. Solche Prozesse spielen eine wesentliche Rolle bei der Hautalterung.

Diese hautpflegenden Wirkstoffe haben, da sie als Antioxidantien wirken, den Nachteil, dass sie durch äußere Einflüsse, insbesondere Lichteinflüsse, mit der Zeit zerstört werden.

Als weiterer Hautpflegewirkstoff ist Ursolsäure oder deren Salze , insbesondere das Natriumsalz, bekannt. Ursolsäure ist ein isolierter Pflanzenwirkstoff, der in der Natur weit verbreitet vorkommt. Es kurbelt den Stoffwechsel der Haut an und wirkt regenerierend. Selbst bei empfindlicher Haut kann Ursolsäure gut verwendet werden. Ursolsäure weist die folgende Struktur auf

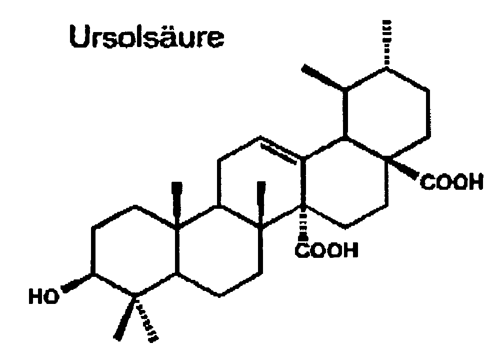

Die Herstellung ist beispielsweise in SU 927249, SU 346300 bzw. deren Derivate in DE 2412355 beschrieben.

Der Einsatz von Ursolsäure in kosmetischen Zubereitungen wird beispielsweise in WO 2002015869, EP 1129697, WO 2001017523, WO 9813019, WO 9800093 und WO 2002053089 oder in der Literatur "Purified Plant Extracts; K. Lintner; Cosmetics&Toiletries Magazine/67 Vol. 113; 1998" beschrieben. Hierin wird die Ursolsäure in Kombination mit verschiedensten bekannten kosmetischen Wirkstoffen wie z.B. Retinol oder Palmitoyl -pentapeptide kombiniert. Die Zubereitungen werden zur Behandlung von Akne, zur Verminderung der Hautalterung, Faltenreduktion etc. eingesetzt.

Ursolsäure stimuliert die Collagensynthese der Haut und verbessert somit die Elastizität der Haut. Dieser Effekt tritt jedoch nicht kurzfristig auf, so dass eine lang andauernde Behandlung mit Ursolsäure erforderlich ist um effektive Wirkungsergebnisse zu erzielen.

US 20020192245 offenbart einen Noni-(morinda citrifolia fruit oder seed)-Extrakt, der zur Pflege der Haut eingesetzt werden kann. Dieser Extrakt wiederum besteht aus oder umfasst eine Vielzahl natürlicher Bestandteile, wovon einer Ursolsäure sein kann.

US 20020028257 offenbart ein Haarpflegeprodukt, das u.a Gingko Biloba und Ursolsäure neben anderen Bestandteilen enthalten kann.

DE 4429928 offenbart ein Mittel zur topischen Hautbehandlung enthaltend eine Wirkstoffkombination aus Ginkgo-Extrakt und Glycin sowie gegegebenfalls ölhaltige Nanosomen, Mono., Di- oder Trihydroxycarbonsäure bzw. deren Reaktionsprodukte mit Proteinen und/oder Polysacchariden und Panthenol.

FR 2699818 offenbart eine kosmetische Zubereitung umfassend Polyphenole und Ginkgo-Extrakte.

Aufgabe der vorliegenden Erfindung ist es die Wirksamkeit von Ursolsäure -haltigen Hautpflegeprodukten zu verbessern.

Zur Hautpflege sind neben klassischer Creme oder Lotion auch Hautauflagen, sogenannte Pads, aus Tuch oder Watte bekannt, die ggf. mit Wirkstofflösung getränkt sind. Darüber hinaus können Wirkstoffe über Pflasterapplikation über so genannte Drug Delivery Systeme (DDS) appliziert werden.
Wirkstoffhaltige Pflaster zählen zu den Drug Delivery Systemen. Es handelt sich um pflasterartige Arznei- oder Kosmetikformen, die den Wirkstoff in Form eines Reservoirs enthalten, aus dem die Wirkstofffreisetzung über einen längeren Zeitraum nach 0. Ordnung erfolgen sollte. Aufgrund dieser Eigenschaften sind die Drug Delivery Systeme in der Lage, den Organismus unabhängig von der Wirkstoffkonzentration im Reservoir über einen langen Zeitraum mit konstanten Mengen Wirkstoff pro Zeiteinheit zu versorgen.

Drug Delivery Systeme werden in die Gruppen Topical und Transdermal Delivery eingeteilt. Die topicalen Formulierungen enthalten Wirkstoffe, deren Freisetzung und Wirkung auf den Bereich direkt unterhalb und der Umgebung des Orts der Applikation begrenzt ist. Die transdermalen Formulierungen hingegen enthalten Wirkstoffe, die durch die Haut appliziert werden, um durch das Eindringen in das Gefäßsystem im gesamten Organismus einen wirksamen Wirkstoffspiegel zu erzeugen.
Darüber hinaus kann die Wirkstoffabgabe über Matrixpflaster über die der Haut abgewandten Seite, an die Umgebungsluft, erfolgen.

Die Drug Delivery Systeme haben gegenüber anderen Applikationsformen (z.B. Salbe, Sprays, Suppositorien, Tabletten) eine Reihe von Vorteilen:
- Aufgrund der langen Wirkungsdauer der Drug Delivery Systeme wird die Compliance der Patienten im Vergleich zu Arzneiformen, die mehrmals im Verlauf eines Tages appliziert werden müssen, deutlich verbessert.
- Durch die Applikation der Drug Delivery Systeme kann eine Senkung der Dosis erfolgen, wodurch eine Verminderung der Nebenwirkungen von Wirkstoffen erreicht wird. Dies ist besonders für Wirkstoffe mit einer geringen therapeutischen Breite von Interesse.
- Beim Auftreten von Nebenwirkungen kann die Applikation des Wirkstoffs durch die Entfernung des Drug Delivery Systems schlagartig gestoppt werden.
- Durch die konstante Freisetzung des Wirkstoffs werden die Schwankungen der Wirkstoffkonzentration, die bei einer wiederholten Applikation am Tag auftreten, im Bereich der Haut und im Serum verhindert.
- Die transdermale Applikation reduziert im Vergleich zu oralen Arzneiformen den first-pass Metabolismus des Wirkstoffs, da der Metabolismus im Bereich der Haut im Vergleich zum Metabolismus im Magen -Darm-Trakt und der Leber deutlich geringer ist. Dies hat zur Folge, dass die Dosis der mit Drug Delivery Systemen applizierten Wirkstoffe im Vergleich zu den oralen Arzneiformen reduziert werden kann.
- Bei den Drug Delivery Systemen muss im Gegensatz zu den oralen Arzneimitteln nicht mit dem Einfluss der Nahrung auf die Penetration des Wirkstoffs gerechnet werden.
- Die Dosierung kann sehr leicht über die Fläche der Drug Delivery Systeme definiert werden.

Auf der anderen Seite weisen die bekannten Drug Delivery Systeme einige Nachteile auf:
- Die Zahl der Wirkstoffe, die mit dieser Darreichungsform ausreichend hohe Blutspiegel oder Konzentrationen im Bereich der Derma erzeugen, um im Organismus einen pharmakologischen Effekt zu erzielen, ist sehr gering.
- Die Wirkung tritt, bedingt durch die zunächst notwendige Hautsättigung mit dem Wirkstoff, erst mit einer gewissen Verzögerung ein.
- Im Bereich der Haut können aufgrund von Bestandteilen der Drug Delivery Systeme allergische Reaktionen ausgelöst werden.
- Drug Delivery Systeme können insbesondere bei längerer Anwendung am gleichen Applikationsort Schäden im Bereich der Haut auslösen. Aus diesem Grund sollten die Drug Delivery Systeme lediglich im Bereich von Hautarealen appliziert werden, die gesund und intakt sind.

Eine Aufgabe der vorliegenden Erfindung ist es daher unter Beibehaltung der Vorteile, die Nachteile der bekannten Drug Delivery Systeme zu vermeiden.

Als kosmetische oder medizinische Zubereitungen sind oftmals Emulsionen, hier insbesondere W/O-, O/W- oder W/O/W-Emulsionen, im Einsatz. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten (W/O) in Form feinster Tröpfchen in der anderen Flüssigkeit dispergiert.
Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-ÖI-Emulsion (W/O-Emulsion, z. B. Butter) handelt es sich um das umgekehrte Prinzip, wobei der Grundcharakter hier durch das Öl bestimmt wird.
In die Emulsionen können öl- als auch wasserlösliche Wirkstoff inkorporiert werden.

Aufgabe der vorliegenden Erfindung ist es kosmetische oder dermatologische Zubereitungen bereit zu stellen, die zur Behandlung von Hautirritationen, Altershaut, Faltenreduktion geeignet sind.

Aufgabe der vorliegenden Erfindung ist es auch eine alternative Applikationsform zu den bekannten Ursolsäure -Zubereitungen zur Verfügung zu stellen.

Gelöst werden die Aufgaben durch ein Hautpflegeprodukt nach Anspruch 1. In den Unteransprüchen sind bevorzugte Ausführungsformen des Produktes offenbart.

Es war überraschend und für den Fachmann nicht vorauszusehen, dass ein Hautpflegeprodukt umfassend 0,01 bis 5 Gew.%Salze der Ursolsäure und 0,0001 bis 10 Gew.% Ginkgo-Extrakt, jeweils bezogen auf die Gesamtmasse des Produktes, wobei als Salze der Ursolsäure das Triethanolamin-Salz, Kalium-Salz, Arginin-Salz, Trisamino-Salz und/oder das Natrium-Salz verwendet werden, den Nachteilen des Standes der Technik abhelfen.

Ginkgo-Extrakte sind als Ginkgo-Biloba Extrakte oder als Herbalia® Ginkgo (CAS 90045-36-6) bekannt. Ginkgo -Biloba ist ein Extrakt aus den Blättern des Frauenhaarfams. Ginkgo Extrakte haben antioxidative Eigenschaften und schützen die Nervenzellen. Ginkgo-Biloba reguliert auch den Tonus und die Elastizität der Blutgefäße positiv, was wiederum eine bessere Zirkulation sowohl in den großen als auch in den Mikrogefäßen bewirkt. Jahrtausend alte Überlieferungen, insbesondere aus dem asiatischen Raum, berichten von der heilenden Wirkung der Ginkgo-Blätter und von deren Wirkstoffen, die die Fließeigenschaften des Blutes und damit die Durchblutung verbessern. Äußerlich angewendet ist Ginkgo eine sanfte Pflege für straffes Gewebe, geschmeidige Haut, glänzendes Haar und zur Behandlung von Pigmentflecken geeignet.
Die arzneiliche Wirkung der Ginkgo -Extrakte beruht dabei vor allem auf den Stoffgruppen der Flavonoide und der Terpenlactone.

In intensiven Studien wurde die erstaunliche Erkenntnis gewonnen, dass die Kombination von 0,01 bis 5 Gew.%Salze der Ursolsäure und 0,0001 bis 10 Gew.% Ginkgo-Extrakt eine synergistische Steigerung der Hautpflegeeigenschaften im Vergleich zu den beiden Einzelbestandteilen verursacht. Bevorzugt ist eine Menge an Gingko -Extrakt im Bereich von 0,001 bis 5 Gew.%. Die Ursolsäure ist aufgrund ihrer Schwerlöslichkeit der Hautpflege über gängige kosmetische Zubereitungen schwer zugänglich. Die Ursolsäure kommt daher häufig in Kombination mit der Oleanolsäure vor, weil es sich bei der Oleanol- und Ursolsäure um strukturverwandten Verbindungen handelt, die technisch nicht zu trennen sind. Die Kombination aus Ursolsäure und Oleanolsäure ist Bestandteil gängiger Handelsprodukte. Bevorzugt werden Mischungen in Bereichen von 60 bis 95 Gew.% Ursol- und 5 bis 40 Gew.% Oleanolsäure, bezogen auf die Gesamtmischungsmasse. Bevorzugt ist ein Mischungsverhältnis von 70 bis 90 Gew.%, insbesondere 80 Gew.%, Ursolsäure zu 10 bis 30 Gew.%, insbesondere 20 Gew.%, Oleanolsäure.

Durch den Zusatz von Ginkgo-Extrakt wird die Wirkung der Salze der Ursolsäure deutlich gesteigert und eine hautpflegende Wirkung ist schon nach kurzer Anwendungszeit merk- und sichtbar. Innerhalb weniger Wochen ist ein statistisch signifikanter Effekt auf die Festigkeit der Haut, auf das Gesichtsvolumen und auf die Hautfalten, besonders in der Augenregion zu beobachten. Vorteilhaft ist, wenn ein mit polaren Lösungsmittel extrahierter Ginkgo-Extrakt verwendet wird. Als besonders geeignet hat sich das erfindungsgemäße Produkt zur Straffung der Gesichtskontur erwiesen.

Die Kombination von Salzen der Ursolsäure und Ginkgo-Extrakt ist verantwortlich für eine synergistische Wirkungsverbesserung. Der Ginkgo-Extrakt wird durchblutungsfördernd und regt die Mikrozirkulation an. In Kombination mit Salzen der Ursolsäure kann diese dann besser und effektiver an den Wirkort in der Haut vordringen.

Als Salze der Ursolsäure werden das Triethanolamin-Salz, Kalium-Salz, Arginin-Salz, Trisamino-Salz und/oder besonders bevorzugt das Natrium-Salz verwendet.

Das Hautpflegeprodukt wird zur Behandlung und/oder Pflege der Haut eingesetzt um die beginnende Hautalterung, die u.a. mit dem Verlust der Elastizität der Haut und dem Entstehen von Fältchen und Falten einhergeht, zu unterdrücken.

Als Hautpflegeprodukt kann die Kombination aus Salzen der Ursolsäure und Ginkgo-Extrakt in verschiedensten Applikationsformen vorliegen.

Beispielsweise ist es möglich die Wirkstoffkombination als drug delivery system, als Pflasterapplikation, bereit zu stellen. Die Wirkstoffe können in die Klebemasse eingearbeitet werden, wie es beispielsweise in der DE 10128685 oder DE 19749467 beschrieben wird,

Des weiteren kann die Wirkstoffkombination als Tränkungslösung für die Tuch- oder Padapplikation vorliegen. Ebenso ist es möglich die Kombinatoin in Aerosol oder Nicht - Aerosolformen zu applizieren.

Vorteilhaft ist, dass das erfindungsgemäße Hautpflegeprodukt als galenische Formulierung bereit gestellt wird. Unter galenischen Formulierungen versteht der Fachmann, Hydrogele, Hydrodispersionsgel, Fettgele, Salben, Emulsionen, Lösungen, Pasten, Kapseln, Öle, Stifte, Suspensionen, Puder, Pulver, Mikroemulsionen, Nanoemulsionen, Wasser, Schüttelemulsionen, Festiger, Lacke oder Rinse-off-Formulierungen. All diese den Fachmann bekannten galenischen Formulierungen erweisen sich für das erfindungsgemäße Produkt als vorteilhaftes Vehikel, um die vorteilhaften Eigenschaften für den Anwender nutzbar zu machen.

Bevorzugt ist das erfindungsgemäße Hautpflegeprodukt als kosmetische oder dermatologische Zubereitung auf Emulsionsbasis. Als Emulsionen kommen insbesondere W/O-, O/W-, S/W-, W/O/W-, O/W/O- oder W/S-Emulsionen zum Einsatz.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, dass die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Erfindung werden Lipide angesehen, deren Grenzflächen -spannung gegen Wasser weniger als 20 mN/m beträgt als unpolar solche, deren Grenzflächenspannung gegen Wasser mehr als 30 mN/m beträgt. Lipide mit einer Grenzflächenspannung gegen Wasser zwischen 20 und 30 mN/m werden im allgemeinen als mittelpolar bezeichnet.

Vorteilhafte polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Besonders vorteilhafte polare Lipide im Sinne der vorliegenden Erfindung sind alle nativen Lipide, wie z. B. Olivenöl, Sonnenblumenöl, Sojaöl, Erdnussöl, Rapsöl, Mandelöl, Palmöl, Kokosöl, Rizinusöl, Weizenkeimöl, Traubenkemöl, Distelöl, Nachtkerzenöl, Macadamianussöl, Maiskeimöl, Avocadoöl und dergleichen sowie die im folgenden aufgelisteten.

| **Handelsname** | **INCI-Name** | **Polarität [mN/m]** |
|---|---|---|
| Isofol 14 T | Butyl Decanol (+) Hexyl Octanol (+) Hexyl Decanol (+) Butyl Octanol | 19,8 |
| Lipovol MOS-130 | Tridecyl Stearate(+) Tridecyl Trimellitate(+) Dipentaerythrityl Hexacaprylate/Hexacaprate | 19,4 |
| Ricinusoel | | 19,2 |
| Isofol Ester 0604 | | 19,1 |
| Miglyol 840 | Propylene Glycol Dicaprylate/Dicaprate | 18,7 |
| Isofol 12 | Butyl Octanol | 17,4 |
| Tegosoft SH | Stearyl Heptanoate | 17,8 |
| Avocadooel | | 14,5 |
| Cetiol B | Dibutyl Adipate | 14,3 |
| Dermol 488 | PEG 2 Diethylenhexanoate | 10,1 |
| Cosmacol ELI | C12-13 Alkyl Lactate | 8,8 |
| Dermol 489 | Diethylen Glycol Dioctanoate(/Diisononanoate | 8,6 |
| Cosmacol ETI | Di-C12/13 Alkyl Tartrate | 7,1 |
| Emerest 2384 | Propylene Glycol Monoisostearate | 6,2 |
| Myritol 331 | Cocoglycerides | 5,1 |
| Prisorine 2041 GTIS | Triisostearin | 2,4 |

Ferner kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase einen Gehalt an C₁₂₋₁₅-Alkylbenzoat aufweist oder vollständig aus diesem besteht.

Ferner kann die Ölphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung 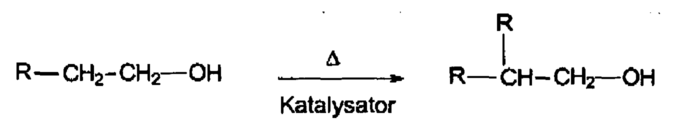 durch Oxidation eines Alkohols zu einem Aldehyd, durch Aldol -Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur 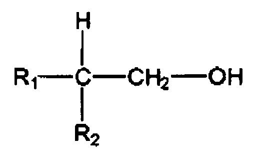 aus. Dabei bedeuten R₁ und R₂ in der Regel unverzweigte Alkylreste.

Erfindungsgemäß vorteilhaft werden der oder die Guerbet-Alkohole gewählt aus der Gruppe, bei denen
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl.

Erfindungsgemäß bevorzugte Guerbet -Alkohole sind das 2-Butyloctanol - es hat die chemische Struktur 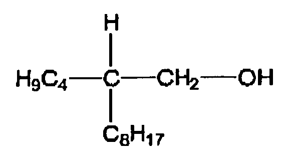 und ist beispielsweise unter der Handelsbezeichnung Isofol^{®} 12 von der Gesellschaft Condea Chemie GmbH erhältlich - und das 2-Hexyldecanol - es hat die chemische Struktur 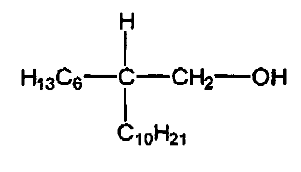 und ist beispielsweise unter der Handelsbezeichnung Isofol® 16 erhältlich. Auch Mischungen von erfindungsgemäßen Guerbet -Alkoholen sind erfindungsgemäß vorteilhaft zu verwenden. Mischungen aus 2-Butyloctanol und 2-Hexyldecanol sind beispielsweise unter der Handelsbezeichnung Isofol® 14 erhältlich.

Die Gesamtmenge an Guerbet-Alkoholen in den fertigen kosmetischen oder dermatologischen Zubereitungen wird vorteilhaft aus dem Bereich bis 25,0 Gew.%, bevorzugt 0,5 - 15,0 Gew.% gewählt, bezogen auf das Gesamtgewicht der Zubereitungen.

Auch beliebige Abmischungen solcher Öl - und Wachskomponenten sind vorteilhaft im Sinne der vorliegenden Erfindung einzusetzen. Es kann auch gegebenenfalls vorteilhaft sein, Wachse, beispielsweise Cetylpalmitat, als Lipidkomponente der Ölphase einzusetzen.

Besonders vorteilhafte mittelpolare Lipide im Sinne der vorliegenden Erfindung sind die im folgenden aufgelisteten Substanzen:

| **Handelsname** | **INCI-Name** | **Polarität [mN/m]** |
|---|---|---|
| DUB VCI 10 | Isodecyl Neopentanoate | 29.9 |
| Dermol IHD | Isohexyldecanoate | 29,7 |
| Dermol 108 | Isodecyl Octanoate | 29,6 |
| Dihexyl Ether | Dihexyl Ether | 29.2 |
| Dermol 109 | Isodecyl 3,5,5 Trimethyl Hexanoate | 29,1 |
| Cetiol SN | Cetearyl Isononanoate | 28,6 |
| Isopropylpalmitat | Isopropylpalmitat | 28,8 |
| Jojobaöl Gold | | 26,2 |
| Wacker AK 100 | Dimethicone | 26,9 |
| Dermol 98 | 2- Ethylhexanosäure 3,5,5 Trimethylester | 26,2 |
| Eutanol G | Octyldodecanol | 24,8 |
| Isofol 16 | Hexyl Decanol | 24,3 |
| Dermol 139 | Isotridecyl 3,5,5 Trimethylhexanonanoate | 24,5 |
| Cetiol PGL | Hexyldecanol (+) Hexyl Decyl Laurate | 24,3 |
| Cegesoft C24 | Octyl Palmitate | 23,1 |
| M.O.D. | Octyldodeceyl Myristate | 22,1 |
| Macadamia Nut Oil | | 22,1 |
| Silikonöl VP 1120 | Phenyl Trimethicone | 22,7 |
| Isocarb 12 | Butyl Octanoicacid | 22,1 |
| Isopropylstearat | Isopropyl Stearate | 21,9 |
| Finsolv TN | C12-15 Alkyl Benzoate | 21,8 |
| Dermofeel BGC | Butylene Glycol Caprylate/Caprate | 21,5 |
| Miglyol 812 | Caprylic/Capric Triglyceride | 21,3 |
| Trivent OCG | Tricaprylin | 20,2 |
| Dermol 866 | PEG ,, Diethylhexanoate/ Diisononanoate/ Ethylhexyl Isononanoate | 20,1 |

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Besonders vorteilhafte unpolare Lipide im Sinne der vorliegenden Erfindung sind die im folgenden aufgelisteten Substanzen:

| **Handelsname** | **INCI-Name** | **Polarität [mN/m]** |
|---|---|---|
| Ecolane 130 | Cycloparaffin | 49,1 |
| Nexbase 2006 FG | Polydecene | 46.7 |
| Polysylane | Hydrogenated Polyisobutene | 44.7 |
| Wacker Silikonöl AK 50 | Polydimethylsiloxan | 46,5 |
| Solvent ICH | Isohexadecane | 43.8 |
| Pionier 2076 | Mineral Oil | 43.7 |
| Pionier 6301 | Mineral Oil | 43.7 |
| Wacker Silikonöl AK 35 | Polydimethylsiloxan | 42,4 |
| Isoeikosan | Isoeikosan | 41.9 |
| Isofol 1212 Carbonat | | 40,3 |
| Softcutol O | Ethoxydiglycol Oleate | 40,5 |
| Lipodermanol OL | Decyl Olivate | 40,3 |
| Cetiol S | Dioctylcyclohexane | 39,0 |
| Pionier 2071 | Mineral Oil | 38.3 |
| Hydrobrite 1000 PO | Paraffinum Liquidum | 37,6 |
| Tegosoft HP | Isocetyl Palmitate | 36,2 |
| Isofol Ester 1693 | | 33,5 |
| Isofol Ester 1260 | | 33,0 |
| Prisorine 2036 | Octyl Isostearate | 31.6 |
| Cetiol CC | Dicaprylyl Carbonate | 31,7 |
| Dermol 99 | Trimethylhexyl Isononanoate | 31,1 |
| Dermol 89 | 2-Ethylhexyl Isononanoate | 31,0 |
| Cetiol OE | Dicaprylyl Ether | 30,9 |
| Dihexylcarbonat | Dihexyl Carbonate | 30,9 |
| Silkflo 366 NF | Polydecene | 30,1 |
| Estol 1540 EHC | Octyl Cocoate | 30.0 |

Es ist jedoch auch vorteilhaft, Gemische aus höher- und niederpolaren Lipiden und dergleichen zu verwenden. So kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montan-wachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), und Syncrowax AW 1C (C₁₈₋₃₈ -Fettsäure) erhältlich sind sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocasfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Erfindungsgemäß können die Fett - und/oder Wachskomponenten sowohl einzeln als auch im Gemisch vorliegen. Auch beliebige Abmischungen solcher Öl- und Wachs-komponenten sind vorteilhaft im Sinne der vorliegenden Erfindung einzusetzen.

Vorteilhaft wird die zusätzliche Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C₁₂₋₁₅-Alkylbenzoat, Capryl-Caprinsäure-triglycerid, Dicaprylylether. Besonders vorteilhaft sind Mischungen aus Octyldodecanol, Capryl-Caprinsäure-triglycerid, Dicaprylylether, Dicaprylyl Carbonat, Cocoglyceriden, oder Mischungen aus C₁₂₋₁₅-Alkybenzoat und 2-Ethylhexylisostearat, Mischungen aus C₁₂₋₁₅-Alkybenzoat und Butylen Glycol Dicaprgat/Dicaprat sowie Mischungen aus C₁₂₋₁₅-Alkybenzoat, 2-Ethylhexylisostearat und Isotridecylisononanoat.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden.

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

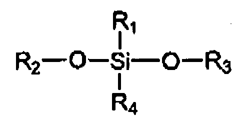

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, PhenylGruppen u. a.) abgesättigt sind.

Als einzusetzenden linearen Silicone mit mehreren Siloxyleinheiten werden im allgemeinen durch Strukturelemente charakterisiert wie folgt: 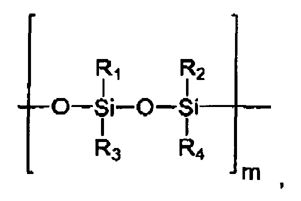 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten Verbindungen dieser Gruppe darstellen und sich durch die folgende Strukturformel auszeichnen 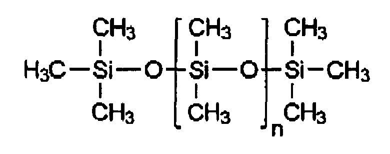 werden auch als Polydimethylsiloxan bzw. Dimethicon (INCI) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

Besonders vorteilhafte Polyorganosiloxane im Sinne der vorliegenden Erfindung sind ferner beispielsweise Dimethylpolysiloxane [Poly(dimethylsiloxan)], welche z. B. unter den

Handelsbezeichnungen ABIL 10 bis 10 000 erhältlich sind. Ferner vorteilhaft sind Phenylmethylpolysiloxane (INCI: Phenyl Dimethicone, Phenyl Trimethicone), aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen erhältlich sind.

Die wäßrige Phase der emulsionsbasierenden Zubereitungen enthält gegebenenfalls vorteilhaft Alkohole, Diole oder Polyole niedriger Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin.

Ein besonderer Vorzug der vorliegenden Erfindung ist es, dass sie gestattet, hohe Konzentrationen an Polyolen, insbesondere Glycerin einzusetzen.

Bevorzugt enthalten Emulsionen gemäß der vorliegenden Idee ein oder mehrere Hydrokolloide.

"Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wässrigen Medien ist. Voraussetzung dafür ist, dass diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

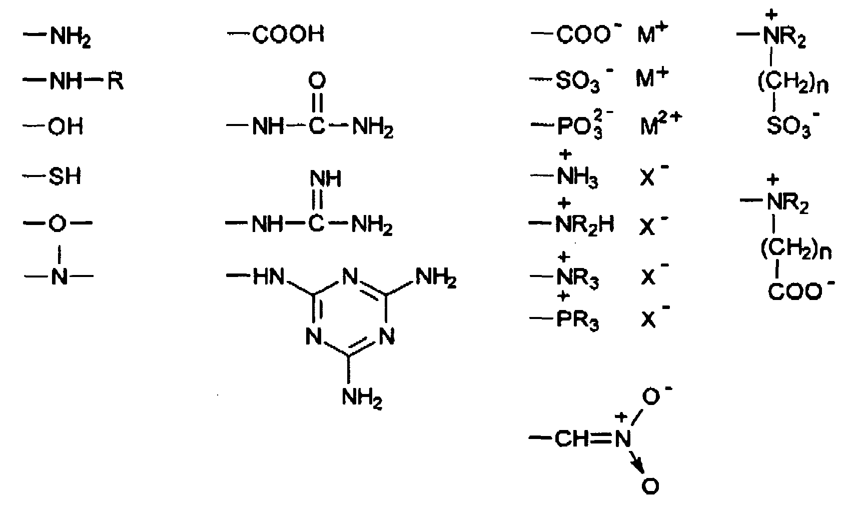

Die Gruppe der kosmetisch und dermatologisch relevanten Hydrokolloide läßt sich wie folgt einteilen in:
- organische, natürliche Verbindungen, wie beispielsweise Agar-Agar, Carrageen, Tragant, Gummi arabicum, Alginate, Pektine, Polyosen, Guar-Mehl, Johannisbrotbaumkemmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide,
- anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus

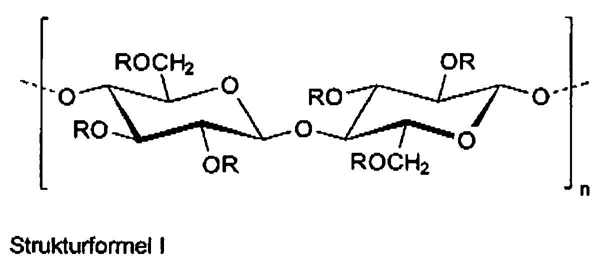

in der R ein Wasserstoff oder eine Methylgruppe darstellen kann.

Insbesondere vorteilhaft im Sinne der vorliegenden Idee sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxy propyl)methylcellulosen, beispielsweise die unter der Handelsbezeichnung Methocel E4M erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder CH2-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Idee ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2x106 bis 24x106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Idee ist ferner Carrageen, ein gelbildender und ähnlich wie Agar aufgebauter Extrakt aus nordatlantischen, zu den Florideen zählenden Rotalgen (Chondrus crispus und Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht lösl. ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt . Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute 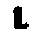 -Carrageenan ist sowohl wasserlöslich als auch gelbildend. Weitere Carrageen-Typen werden ebenfalls mit griechischen Buchstaben bezeichnet: α, β, γ, µ, ν, ξ, π, ω, χ. Auch die Art vorhandener Kationen (K+, NH4+, Na+, Mg2+, Ca2+) beeinflusst die Löslichkeit der Carrageene.

Die Verwendung von Chitosan in kosmetischen Zubereitungen ist per se bekannt. Chitosan stellt ein partiell deacyliertes Chitin dar. Dieses Biopolymer hat u.a. filmbildende Eigenschaften und zeichnet sich durch ein seidiges Hautgefühl aus. Von Nachteil ist jedoch seine starke Klebrigkeit auf der Haut, die insbesondere - vorübergehend - während der Anwendung auftritt.

Entsprechende Zubereitungen können dann im Einzelfalle nicht vermarktungsfähig sein, da sie vom Verbraucher nicht akzeptiert bzw. negativ beurteilt werden. Chitosan wird bekanntermaßen beispielsweise in der Haarpflege eingesetzt. Es eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H.P.Fiedler, "Lexikon der Hilfsstoffe für Pharmazie, Kosmetik und angrenzende Gebiete", dritte Auflage 1989, Editio Cantor, Aulendorf, S. 293, Stichwort "Chitosan".

Chitosan ist gekennzeichnet durch folgende Strukturformel: 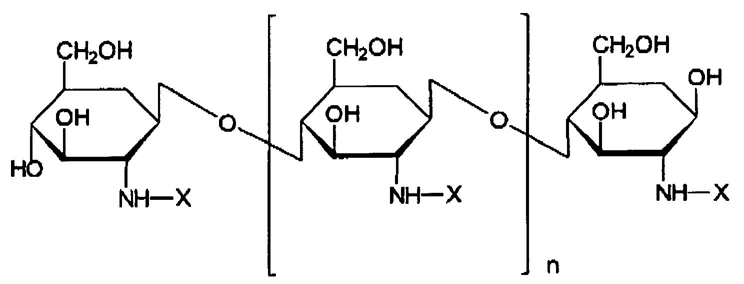 dabei nimmt n Werte bis zu ca. 10.000 an, X stellt entweder den Acetylrest oder Wasserstoff dar Chitosan entsteht durch Deacetylierung und teilweise Depolymerisation (Hydrolyse) von Chitin, welches durch die Strukturformel 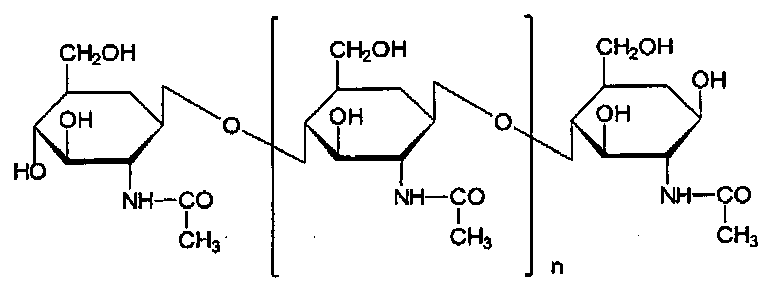 gekennzeichnet ist. Chitin ist wesentlicher Bestandteil des Ektoskeletts ['o χ 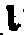 των= grch.: der Panzerrock] der Gliederfüßer (z.B. Insekten, Krebse, Spinnen) und wird auch in Stützgeweben anderer Organismen (z.B. Weichtiere, Algen, Pilze) gefunden.

Im Bereich von etwa pH <6 ist Chitosan positiv geladen und dort auch in wäßrigen Systemen löslich. Es ist nicht kompatibel mit anionischen Rohstoffen. Daher bietet sich zur Herstellung chitosanhaltiger Öl-in-Wasser-Emulsionen der Einsatz nichtionischer Emulgatoren an. Diese sind an sich bekannt, beispielsweise aus der EP-A 776 657.

Erfindungsgemäß bevorzugt sind Chitosane mit einem Deacetylierungsgrad > 25 % , insbesondere > 55 bis 99 % [bestimmt mittels 1H-NMR]).

Es ist von Vorteil, Chitosane mit Molekulargewichten zwischen 10.000 und 1.000.000 zu wählen, insbesondere solches mit Molekulargewichten zwischen 100.000 und 1.000.000. [bestimmt mittels Gelpermetionschromatographie].

Polyacrylate sind ebenfalls vorteilhaft im Sinne der vorliegenden Idee zu verwendende Gelatoren. Erfindungsgemäß vorteilhafte Polyacrylate sind Acrylat -Alkylacrylat-Copolymere, insbesondere solche, die aus der Gruppe der sogenannten Carbomere oder Carbopole (Carbopol® ist eine eingetragene Marke der B. F. Goodrich Company) gewählt werden. Insbesondere zeichnen sich das oder die vorteilhaften Acrylat-Alkylacrylat-Copolymere durch die folgende Struktur aus:

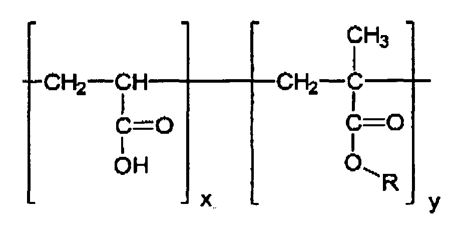

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat -Copolymere und/oder Acrylat-Alkylacrylat-Copolymere, welche unter den Handelbezeichnungen Carbopol® 1382, Carbopol® 981 und Carbopol® 5984 erhältlich sind.

Ferner vorteilhaft sind Copolymere aus C10-30-Alkylacrylaten und einem oder mehreren Monomeren der Acrylsäure, der Methacrylsäure oder deren Ester, die kreuzvemetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 erhältlich.

Die Gesamtmenge an einem oder mehreren Hydrokolloiden wird in den fertigen kosmetischen oder dermatologischen Zubereitungen vorteilhaft kleiner als 1,5 Gew.%, bevorzugt zwischen 0,1 und 1,0 Gew.%, bezogen auf das Gesamtgewicht der Zubereitungen, gewählt.

Hydrokolloide aus der Gruppe der anionischen Polymere werden vorteilhaft im Sinne der vorliegenden Idee gewählt aus der Gruppe der Carbomere als Natrium-, Kalium-, TEA- und Trisamino-Salze, Natrium-, Kaliumhyaluronat, Microcrystalline Cellulose + Cellulose Gum, Veegum-Typen, Hektorite, Bentonite, Laponite, Alginate, Methacrylate.

Hydrokolloide aus der Gruppe der nichtionischen Polymere werden vorteilhaft im Sinne der vorliegenden Idee gewählt aus der Gruppe Polyvinylpyrolidon, Hydroxypropyl Methylcellulose, Polyvinylalkohol, Polyether-1, Xanthan Gum, Hydroxyethylcellulose, Cellulosederivate, Stärke, Stärkederivate, Guar Gum, Glycerylmethacrylat.

Hydrokolloide aus der Gruppe der kationischen Polymere werden vorteilhaft im Sinne der vorliegenden Idee gewählt aus der Gruppe Chitosan, kationische Stärkederivate, kationische Cellulosederivate, Guar-Hydroxypropyltrimethylammoniumchlorid, Natriumpolystyrolsulfonat.

Besonders vorteilhafte Zubereitungen werden ferner erhalten, wenn als weitere Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L -Carnosin, D-Carnosin, L-Carnosin und deren Derivate (z.B. Anserin), Carotinoide, Carotine (z.B. α-Carotin, β-Carotin, Ψ-Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiadipropianat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat). Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, Trans-Stilbenoxid) und die geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Idee können öllösliche Antioxidantien eingesetzt werden.

Eine erstaunliche Eigenschaft der vorliegenden Erfindung ist, dass das hautpflegeprodukt, insbesondere als Zubereitungen, sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.
Die Menge der Antioxidantien (eine oder mehrere Verbindungen) in den Zubereitungen beträgt vorzugsweise 0,001 bis 30 Gew.%, besonders bevorzugt 0,05 - 20 Gew.%, insbesondere 1 - 10 Gew.%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.%, bezogen auf das Gesamtgewicht der Formulierung, zu wählen.

Sofern Vitamin A, bzw. Vitamin -A-Derivate, bzw. Carotine bzw. deren Derivate das oder die Antioxidantien darstellen, ist vorteilhaft, deren jeweilige Konzentrationen aus dem Bereich von 0,001 - 10 Gew.%, bezogen auf das Gesamtgewicht der Formulierung, zu wählen.

Es ist dem Fachmann natürlich bekannt, dass kosmetische Zubereitungen zumeist nicht ohne die üblichen Hilfs- und Zusatzstoffe denkbar sind. Die kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Filmbildner, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide. Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Vorteilhafte anfeuchtende bzw. feuchthaltende Mittel (sogenannte Moisturizer) im Sinne der vorliegenden Idee sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registratumummer 178463-23-5 abgelegt und z. B. unter der Bezeichnung Fucogel®1000 erhältlich ist.

Filmbildner im Sinne der vorliegenden Idee sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen eine Schutzfunktion hat.

Es ist insbesondere von Vorteil, die Filmbildner aus der Gruppe der Polymere auf Basis von Polyvinylpyrrolidon (PVP) 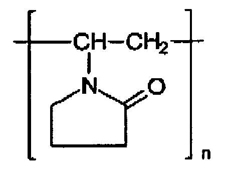 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Es ist insbesondere vorteilhaft, W/O-Pickering-Emulsionen durch die Zugabe von Copolymeren des Polyvinylpyrrolidons zusätzlich zu stabilisieren.

Ebenfalls vorteilhaft sind weitere polymere Filmbildner, wie beispielsweise Natriumpolystrytrertsulfonat, welches unter der Handelsbezeichnung Flexan 130 erhältlich ist, und/oder Polyisobuten, erhältlich unter der Handelsbezeichnung Rewopal PIB1000.

Die Gesamtmenge an einem oder mehreren Filmbildnern wird in den fertigen kosmetischen oder dermatologischen, beispielsweise W/O-Pickering-Emulsionen vorteilhaft kleiner als 10,0 Gew.-%, bevorzugt zwischen 1,0 und 7,0 Gew.-%, bezogen auf das Gesamtgewicht der Zubereitungen, und in O/W-Pickering-Emulsionen vorteilhaft kleiner als 20,0 Gew.-%, bevorzugt zwischen 2,0 und 15,0 Gew.-%, bezogen auf das Gesamtgewicht der Zubereitungen, gewählt.

In den nachfolgenden Beispielen sind verschiedenen erfindungsgemäßen Zubereitungen und deren Verwendung dargestellt. Die angegebenen Anteilswerte sind Gewichtsprozente bezogen auf die Gesamtmasse der Zubereitung , sofern nichts anderes angegeben ist.

### Beispiele

### W/O-Emulsionen mit Ursolsäure und Ginkgo Biloba

| **Beispiel** | **1** | **2** | **3** | **4** | **5** |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | --- | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0.3 | 1,0 |
| Natriumcitrat | 0,2 | 0.05 | 0.4 | 0.3 | 2.0 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2.5 | 3,0 | 5,0 | 0,5 |
| Kaliumsorbat | 0,04 | 0,15 | 0,05 | 0,03 | 0,4 |
| Benzylalkohol | 0,3 | 0,4 | 0,25 | 0,15 | --- |
| Ginkgo Biloba Extract | 0,1 | 0,5 | 1,0 | 2,5 | 10 |
| Ursolsäure; Na-Salz | 0,1 | 0,5 | 0,75 | 1,0 | 0,3 |
| Wasser | ad 100 | ad 100 | ad 100 | ad 100 | ad 100 |

### W/O-Emulsionen mit Ursolsäure und Ginkgo Biloba

| **Beispiel** | **6** | **7** | **8** | **9** | **10** |
|---|---|---|---|---|---|
| PEG-30 Dipolyhydroxystearat | --- | 0,5 | 0,25 | --- | 3,0 |
| Lanolin Alcohol | 1,0 | 1,5 | 1,75 | 3.0 | --- |
| Paraffinöl | 12,5 | 10,0 | 8,0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0.5 | 1,0 | 0,75 | 0,25 | 0,1 |
| Magnesiumsulfat | 0,5 | 0.6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s. |
| 1,3 Butylenglykol | 2,0 | --- | 5,0 | --- | --- |
| Capryl-/Caprinsäuretriglycerid | 2.0 | 2,5 | 3,0 | 5,0 | 0,5 |
| Natriumdehydracet | --- | --- | 0,05 | --- | --- |
| Kaliumsorbat | 0,3 | 0,4 | 0,25 | 0,15 | --- |
| Ginkgo Biloba Extract | 0,1 | 0,5 | 1,0 | 2,5 | 10 |
| Ursolsäure; Na-Salz | 0,1 | 0,5 | 0,75 | 1,0 | 0,3 |
| Talkum | --- | --- | 0,05 | --- | 0,1 |
| Wasser | ad 100 | ad 100 | ad 100 | ad 100 | ad 100 |

### W/S-Emulsion mit Ursolsäure und Ginkgo Biloba

| **Beispiel** | **11** | **12** | **13** | **14** | **15** |
|---|---|---|---|---|---|
| Cetyl PEG/PPG-10/1 Dimethicone | 1,0 | --- | --- | 3,0 | 5,0 |
| Cylomethicon + PEG/PPG-18/18 Dimethicon (90:10) | 10,0 | 12,5 | 25 | --- | --- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1.5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s, | q,s, | q,s, | q,s, |
| Kaliumsorbat | 0,4 | 0,1 | 0,05 | 0,3 | 0,4 |
| Cetyldimethicon | 0,5 | --- | 0,7 | --- | --- |
| Benzylalkohol | --- | --- | 0,05 | --- | 0.1 |
| Ginkgo Biloba Extract | 0,1 | 0.5 | 1,0 | 2,5 | 10 |
| Ursolsäure; Na-Salz | 0,1 | 0,5 | 0,75 | 1,0 | 0,3 |
| Wasser | ad 100 | ad 100 | ad 100 | ad 100 | ad 100 |

### W/S-Emulsionen mit Ursolsäure und Ginkgo Biloba

| **Beispiel** | **16** | **17** | **18** | **19** | **20** |
|---|---|---|---|---|---|
| Cetyl PEG/PPG-10/1 Dimethicone | 1,0 | --- | --- | 3,0 | 5,0 |
| Cylomethicon + PEG/PPG-18/18 Dimethicon (90:10) | 10,0 | 12,5 | 25 | --- | --- |
| Cyclomethicon | 12,5 | 15 | 28,0 | 25,0 | 17,5 |
| Dimethicon | 5,0 | 13,0 | 5,0 | 12.0 | 15,0 |
| hydriertes Polyisobuten | 0,5 | 0,75 | 1,0 | 2,0 | 0.25 |
| Octyldodecanol | 0,5 | 1,0 | 0,75 | 3,0 | 0,25 |
| Panthenol | 0,5 | 1,0 | 0,75 | 0.25 | 0,1 |
| Natriumchlorid | 2,0 | 0,6 | 2,5 | 0,7 | 1.0 |
| Glycerin | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s, | q,s, | q,s, | q,s, | q,s, |
| Ginkgo Biloba Extract | 0,1 | 0,5 | 1,0 | 2,5 | 10 |
| Ursolsäure; Na-Salz | 0,1 | 0,5 | 0,75 | 1,0 | 0,3 |
| Stearyldimethicon | 0,5 | --- | 0,7 | --- | --- |
| Dehydracetsäure | --- | --- | 0,05 --- | --- | 0,1 |
| modifizierte Stärke | --- | 2,5 | --- | 0,15 | --- |
| Wasser | ad 100 | ad 100 | ad 100 | ad 100 | ad 100 |

### W/O-Emulsionen mit Ursolsäure und Ginkgo Biloba

| **Beispiel** | **21** | **22** | **23** | **24** | **25** |
|---|---|---|---|---|---|
| PEG-22 Dodecyl Glycol Copolymer | 5,0 | 1,5 | 0,25 | --- | 3,0 |
| PEG-45 Dodecyl Glycol Polymer | 1,0 | 1,5 | 1,75 | 3,0 | --- |
| Paraffinöl | 12,5 | 10.0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Nachtkerzenöi | 0,5 | 0,75 | 1,0 | 2,0 | 0,25 |
| Octyldodecanol | 0,5 | 1,0 | 0,75 | 3,0 | 0,25 |
| Aluminiumstearat | 0.4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Ginkgo Biloba Extract | 0,1 | 0,5 | 1,0 | 2.5 | 10 |
| Ursolsäure; Na-Salz | 0,1 | 0,5 | 0,75 | 1,0 | 0.3 |
| Magnesiumsulfat | 0,5 | 0,6 | 0,5 | 0,7 | 1,0 |
| Glycerin | 3,0 | 5,0 | 10,0 | 15,0 | 1,5 |
| Natriumcitrat | 0,2 | 0,1 | --- | --- | --- |
| Zitronensäure | 0,2 | 0,1 | --- | --- | --- |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Benzylalkohol | --- | --- | 0,05 | --- | 0,1 |
| Wasser | ad 100 | ad 100 | ad 100 | ad 100 | ad 100 |

### W/O-Emulsionen mit Ursolsäure und Ginkgo Biloba

| **Beispiel** | **26** | **27** | **28** | **29** | **30** |
|---|---|---|---|---|---|
| Polyglyceryl-2-dipolyhydroxystearat | 3,0 | --- | 0,25 | --- | 3,0 |
| Polyglyceryl-3 diisostearate | 1,0 | 3,5 | 1,75 | 2,5 | --- |
| PEG-40 sorbitanisostearate | --- | 2,5 | 0.5 | 3.5 | 3,0 |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Isopropylpalmitat | 0,5 | 1,0 | 0.75 | 3,0 | 0,25 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Ginkgo Biloba Extract | 0,1 | 0,5 | 1,0 | 2,5 | 10 |
| Ursolsäure; Na-Salz | 0,1 | 0,5 | 0,75 | 1,0 | 0,3 |
| Magnesiumsulfat | 10,5 | 0,6 | 0,5 | 0,7 | 1,0 |
| Glycerin | 3,0 | 5,0 | 10.0 | 15,0 | 1,5 |
| Zitronensäure | 0,2 | 0,1 | 0,1 | 0.3 | 1,0 |
| Natriumcitrat | 0,2 | 10,3 | 0,2 | 1,5 | 0.8 |
| Parfum | q,s, | q,s, | q,s, | q,s, | q,s, |
| Capryl-/Caprinsäuretriglycerid | 2,0 | 2,5 | 3,0 | 5,0 | 0,5 |
| Kaliumsorbat | 0,24 | 0,15 | 0,05 | 0,3 | 0,4 |
| Wasser | ad 100 | ad 100 | ad 100 | ad 100 | ad 100 |

### Silikon in Wasser-Emulsion mit Ursolsäure und Ginkgo Biloba

| **Beispiel** | **31** | **32** | **33** | **34** | **35** |
|---|---|---|---|---|---|
| Dimethiconcopolyol, Caprylic/Caprictriglycerid | 1,0 | 2,0 | 8,0 | 3,0 | 5,0 |
| Cyclomethicon | 12,5 | 15 | 25,0 | 10,0 | 7,5 |
| Dimethicon | 5,0 | 15,0 | 5,0 | 12,0 | 15,0 |
| Mineralöl | 0,5 | 0,75 | 1,0 | 2,0 | 0,25 |
| Phenyltrimethicon | 0,5 | 1,0 | 0,75 | 3,0 | 0,25 |
| Glycerin | 5,0 | 7,5 | 10,0 | 3,0 | 1,0 |
| Ginkgo Biloba Extract | 0,1 | 0,5 | 1,0 | 2,5 | 10 |
| Ursolsäure: Na-Salz | 0,1 | 0,5 | 0,75 | 1,0 | 0,3 |
| Xanthan Gum | --- | 0,1 | --- | 0,25 | 1,0 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Wasser | ad 100 | ad 100 | ad 100 | ad 100 | ad 100 |

### O/W-Emulsion mit Ursolsäure und Ginkgo Biloba

| **Beispiel** | **36** | **37** | **38** | **39** | **40** |
|---|---|---|---|---|---|
| Glycerylsterat | 1,0 | --- | --- | 0,5 | 0,25 |
| Polyethylenglycol(40)stearat | 10.0 | --- | 5 | --- | --- |
| Triglycerinmethylglucosedistearat | --- | 5,5 | --- | --- | 2,5 |
| Sorbitanstearat | --- | 1.5 | 3 | --- | --- |
| Cyclomethicon | 2,5 | 15 | 8,0 | 5,0 | 7,5 |
| Dimethicon | 5,0 | 3,0 | 5.0 | 2.0 | 5.0 |
| Behenylalkohol | 1 | --- | 2 | 1 | --- |
| Stearylalkohol | --- | 1 | --- | 1 | --- |
| Ginkgo Biloba Extract | 0,1 | 0,5 | 1,0 | 2,5 | 10 |
| Ürsolsäure; Na-Salz | 0,1 | 0,5 | 0,75 | 1,0 | 0,3 |
| Cetylstearylalkohol | --- | --- | 1 | 1 | --- |
| hydriertes Polyisobuten | 0,5 | 0.75 | 1,0 | 2,0 | 0,25 |
| Octyldodecanol | 0,5 | 1,0 | 0,75 | 3.0 | 0,25 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0.4 | 0,25 | 0,15 | --- |
| lodopropynylbutylcarbamat | --- | --- | 0,05 | --- | 0,1 |
| Glycerin | 5 | 10 | 3 | 15 | 7,5 |
| modifizierte Stärke | --- | 2,5 | --- | 0,15 | --- |
| Wasser | ad 100 | ad 100 | ad 100 | ad 100 | ad 100 |

### O/W-Emulsion mit Ursolsäure und Ginkgo Biloba

| **Beispiel** | **41** | **42** | **43** | **44** | **45** |
|---|---|---|---|---|---|
| Polyethylenglycol(21)stearylether | 1 | --- | 2,5 | 2 | 1,5 |
| Polyethylenglycol(2)stearylether | 1 | --- | 5,5 | 3 | 7,5 |
| Cetearylglucosid | --- | 8 | --- | --- | --- |
| Behenylalkohol | 3 | 2 | --- | 1 | --- |
| Stearylalkohol | 3 | 2 | --- | 2 | --- |
| Cetylstearylalkohol | 3 | 4 | --- | --- | 2 |
| hydriertes Polyisobuten | 0,5 | 0,75 | 1,0 | 2,0 | 0,25 |
| Ginkgo Biloba Extract | 0,1 | 0,5 | 1,0 | 2,5 | 10 |
| Ursolsäure; Na-Salz | 0,1 | 0,5 | 0,75 | 1,0 | 0,3 |
| Octyldodecanol | 0.5 | 1,0 | 0,75 | 3,0 | 0,25 |
| Glycerin | 5 | 10 | 15 | 3 | 7,5 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Iodopropynylbutylcarbamat | --- | --- | 0,05 | --- | 0,1 |
| modifizierte Stärke | 0,5 | --- | --- | 0,15 | --- |
| Wasser | ad 100 | ad 100 | ad 100 | ad 100 | ad 100 |

### O/W-Emulsion mit Ursolsäure und Ginkgo Biloba

| **Beispiel** | **46** | **47** | **48** | **49** | **50** |
|---|---|---|---|---|---|
| Glycerylsteratcitrat | 1,0 | 0,5 | 0,1 | 0,5 | 0,3 |
| Polyethylenglycol(20)cetearylether | 10,0 | 1,0 | 5 | --- | --- |
| Triglycerinmethylglucosedistearat | --- | --- | --- | --- | 2,5 |
| Ginkgo Biloba Extract | 0,1 | 0,5 | 1,0 | 2,5 | 10 |
| Ursolsäure; Na-Salz | 0,1 | 0,5 | 0,75 | 1,0 | 0,3 |
| Dimethicon | 0,5 | 3,0 | 0,75 | 1,5 | 0,2 |
| Behenylalkohol | 1 | --- | 2 | 1 | 0,2 |
| Dicaprylylcarbonat | 3 | 5 | 10 | 15 | 5 |
| Stearylalkohol | --- | --- | --- | 1 | 0,2 |
| Cetylstearylalkohol | --- | --- | 1 | 1 | 0,2 |
| Tocopherol | 0,5 | 0,5 | 0.75 | 0,25 | 0,1 |
| Octyldodecanol | 0,5 | --- | 0.75 | 3,0 | 0,25 |
| Panthenol | 0,5 | --- | 0,75 | 0.25 | 0,1 |
| Carbomer | 0,05 | 0,35 | 0,15 | 0,1 | --- |
| Parfum | q,s, | q,s, | q,s, | q,s, | q,s, |
| Caprylic/Capric Triglycerid | 1 | 5 | 3 | 5 | 10 |
| Methylparaben | 0,4 | 0,3 | 0,05 | 0,3 | 0,4 |
| Propylparaben | 0,3 | --- | 0,25 | 0,15 | --- |
| lodopropynylbutylcarbamat | --- | --- | 0,05 | --- | 0,1 |
| Phenoxyethanol | --- | 0,5 | --- | 0,15 | --- |
| Sorbitol | 10 | --- | --- | 5 | --- |
| Butylenglykol | | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

### O/W-Emulsion mit Ursolsäure und Ginkgo Biloba

| **Beispiel** | **51** | **52** | **53** | **54** | **55** |
|---|---|---|---|---|---|
| Glycerylsterat | 2,6 | --- | --- | 2,7 | 2,6 |
| Caprylic/Capric Triglycerid | 3,5 | 4,0 | 3,75 | 3.1 | 5,5 |
| Dicaprylylcarbonat | 3.5 | 2,5 | 3,75 | --- | 3,0 |
| Triglycerinmethylglucosedistearat | --- | 3.0 | 3,0 | --- | --- |
| Cetystearylalkohol | 3,0 | --- | --- ---- | 4,0 | 3,5 |
| Dimethicon | --- | 3,0 | 5,0 | 3,0 | 2,75 |
| Cetylalkohol | --- | 3,0 | 1,5 | 1,25 | --- |
| Polyethylenglycol(40)stearat | 0,8 | --- | --- | 1,3 | 1,3 |
| C12-15 Alkylbenzoat | --- | 2.5 | 3,75 | 3,0 | 4,5 |
| Ginkgo Biloba Extract | 0,75 | 0.75 | 1,0 | 0,5 | 1,0 |
| Ursolsäure ; Na-Salz | 0,3 | 0,3 | 0,45 | 0,125 | 0,3 |
| Natriumoleanolat | 0,1 | 0,1 | 0,1 | 0,075 | 0.1 |
| Cyclomethicon | 3,5 | --- | --- | --- | 0,25 |
| Propylenglykol | 0,5 | 0,5 | 0,5 | 0,35 | 0,2 |
| Mineralöl | --- | --- | --- | 6,0 | --- |
| Phenoxyethanol | 0,4 | 0,4 | 0,4 | 0,75 | 1,25 |
| Zitronensäure | --- | --- | 0,05 | --- | --- |
| Ethanol | --- | --- | --- | 3,0 | --- |
| Ethylparaben | --- | --- | --- | 0,3 | --- |
| Methylparaben | 0,2 | 0,2 | 0,2 | 0,2 | 0,2 |
| Glycerin | 10,0 | 10,0 | 10,0 | 10,0 | 12,5 |
| Propylparaben | 0,1 | 0,1 | 0,1 | 0,1 | 0,1 |
| Carbomer, Natrium-Salz | 0,1 | 0,1 | 0,1 | 0,2 | 0,1 |
| Parfum | q,s, | q,s, | q,s, | q,s, | q,s, |
| Iodopropynylbutylcarbamat | 0,018 | 0,018 | 0,018 | --- | 0,018 |
| Wasser | ad 100 | ad 100 | ad 100 | ad 100 | ad 100 |

## Patentansprüche

1. Hautpflegeprodukt umfassend 0,01 bis 5 Gew.% Salze der Ursolsäure und 0,0001 bis 10 Gew.% Ginkgo-Extrakt, jeweils bezogen auf die Gesamtmasse des Produktes, **dadurch gekennzeichnet, dass** als Salze der Ursolsäure das Triethanolamin-Salz, Kalium-Salz, Arginin-Salz, Trisamino-Salz und/oder das Natrium-Salz verwendet wird.

2. Hautpflegeprodukt nach Anspruch 1, **dadurch gekennzeichnet, dass** mit polaren Lösungsmittel extrahierter Ginkgo-Extrakt verwendet wird.

3. Hautpflegeprodukt nach einem der vorstehenden Ansprüche, **dadurch gekennzeichnet, dass** das Produkt eine galenische Formulierung darstellt.

4. Hautpflegeprodukt nach einem der vorstehenden Ansprüche, **dadurch gekennzeichnet, dass** das Produkt eine emulsionsbasierende kosmetische und/oder dermatologische Zubereitung ist.

5. Hautpflegeprodukt nach einem der Ansprüche 1 bis 3, **dadurch gekennzeichnet, dass** das Produkt ein transdermales therapeutisches Pflaster ist.

6. Hautpflegeprodukt nach einem der Ansprüche 1 bis 3, **dadurch gekennzeichnet, dass** das Produkt ein Aerosol- oder Non-Aerosol-Spray ist.

7. Hautpflegeprodukt nach einem der Ansprüche 1 bis 3, **dadurch gekennzeichnet, dass** das Produkt ein kosmetisches Pad, Tuch oder Papier ist.

## Claims

1. Skincare product comprising 0.01 to 5% by weight of salts of ursolic acid and 0.0001 to 10% by weight of gingko extract, in each case based on the total mass of the product, **characterized in that** the salts of ursolic acid used are the triethanolamine salt, potassium salt, arginine salt, trisamino salt and/or the sodium salt.

2. Skincare product according to Claim 1, **characterized in that** gingko extract extracted using polar solvents is used.

3. Skincare product according to one of the preceding claims, **characterized in that** the product is a galenical formulation.

4. Skincare product according to one of the preceding claims, **characterized in that** the product is an emulsion-based cosmetic and/or dermatological preparation.

5. Skincare product according to one of Claims 1 to 3, **characterized in that** the product is a transdermal therapeutic plaster.

6. Skincare product according to one of Claims 1 to 3, **characterized in that** the product is an aerosol or nonaerosol spray.

7. Skincare product according to one of Claims 1 to 3, **characterized in that** the product is a cosmetic pad, wipe or paper.

## Revendications

1. Produit de soin de la peau, comprenant 0,01 à 5% en poids d'acide ursolique et 0,0001 à 10% en poids d'extrait de ginkgo, à chaque fois par rapport à la masse totale du produit, **caractérisé en ce qu'**on utilise comme sels de l'acide ursolique le sel de la triéthanolamine, le sel de potassium, le sel d'arginine, le sel de trisamino et/ou le sel de sodium.

2. Produit de soin de la peau selon la revendication 1, **caractérisé en ce qu'**on utilise de l'extrait de ginkgo extrait avec du solvant polaire.

3. Produit de soin de la peau selon l'une quelconque des revendications précédentes, **caractérisé en ce que** le produit est une formulation galénique.

4. Produit de soin de la peau selon l'une quelconque des revendications précédentes, **caractérisé en ce que** le produit est une préparation cosmétique et/ou dermatologique à base d'une émulsion.

5. Produit de soin de la peau selon l'une quelconque des revendications 1 à 3, **caractérisé en ce que** le produit est un pansement thérapeutique transdermique.

6. Produit de soin de la peau selon l'une quelconque des revendications 1 à 3, **caractérisé en ce que** le produit est un spray en aérosol ou un spray qui n'est pas aérosol.

7. Produit de soin de la peau selon l'une quelconque des revendications 1 à 3, **caractérisé en ce que** le produit est un tampon, une lingette ou un papier cosmétique.
